# EUROPEAN PATENT APPLICATION

(11) **EP 3 654 342 A1**
(43) Date of publication of application: **20.05.2020**
(21) Application number: 18205898.2
(22) Date of filing: 13.11.2018
(51) Int. Cl.: G16H 20/40

(54) **SYSTEM FOR ASSISTING IN PROVIDING TEMPLATE TREATMENT PARAMETERS FOR ABLATION TREATMENT**

(71) Applicant: Koninklijke Philips N.V., 5656 AG Eindhoven (NL)
(72) Inventor: HAUTVAST, Guillaume Leopold Theodorus, 5656 AE Eindhoven (NL); KUSTRA, Jacek Lukasz, 5656 AE Eindhoven (NL); VAN DIJK, Edmond, 5656 AE Eindhoven (NL); ELEVELT, Aaldert Jan, 5656 AE Eindhoven (NL); BINNEKAMP, Dirk, 5656 AE Eindhoven (NL)
(74) Representative: de Haan, Poul Erik

(57) **Abstract**

It is an object of the present invention to provide a system allowing to provide improved template treatment parameters for ablation treatment allowing an optimized ablation treatment. A system for assisting in providing template treatment parameters is presented. The system comprises a treatment parameter providing unit 101 providing treatment parameters, a patient data providing unit 102 providing technical patient outcome data and/or clinical patient outcome data, a template treatment parameter determining unit 104 for determining template treatment parameters based on a relation between any of the treatment parameters, the technical patient outcome data and/or the clinical patient outcome data. Thus, the template treatment parameters can be optimized in accordance with the technical and/or clinical outcome of the treatment applied using the treatment parameters. Accordingly, the template parameters for treating a patient can be improved and the improved template treatment parameters allow to provide an optimized treatment to a patient.

## Description

### FIELD OF THE INVENTION

The invention relates to a system, a method and a computer program for assisting in providing template treatment parameters for ablation treatment.

### BACKGROUND OF THE INVENTION

The use of thermal ablation using heat or cold for ablating a tissue region constantly increases. For such a thermal ablation therapy, a plurality of treatment parameters like, for instance, device setting parameters, have to be considered and determined by a physician before the application of the treatment. When determining the treatment parameters for each patient, physicians often base their decisions on their own experience and on treatment parameters of past treatments. But, it is often difficult for a physician to get a good overview over the past treatments and to evaluate the efficiency of the used treatment parameters. It would therefore be very helpful to provide template treatment parameters that allow an optimized treatment for each patient.

### SUMMARY OF THE INVENTION

It is an object of the present invention to provide a system, a method and a computer program allowing to provide improved template treatment parameters for ablation treatment allowing an optimized ablation treatment for a patient.

In a first aspect of the present invention a system for assisting in providing template treatment parameters for ablation treatment is presented, wherein the system comprises a) a treatment parameter providing unit adapted to provide treatment parameters, wherein the treatment parameters comprise parameters defining an ablation treatment of a patient, b) a patient data providing unit adapted to provide patient data of a plurality of patients treated with the treatment parameters, wherein the patient data comprises technical patient outcome data defining the technical outcome of the treatment and/or clinical patient outcome data defining the clinical outcome of the treatment, c) a template treatment parameter determining unit adapted to determine template treatment parameters, wherein the template treatment parameters are determined based on a relation between any of the treatment parameters, the technical patient outcome data and/or the clinical patient outcome data.

Since the template treatment parameter determining unit determines template treatment parameters based on a relation between any of the treatment parameters, the technical patient outcome data and/or the clinical patient outcome data, the template treatment parameters can be optimized in accordance with the technical and/or clinical outcome of the treatment applied using the treatment parameters. Thus, the template treatment parameters can be based on the information of past treatments of a plurality of patients. Moreover, since every new treatment provides new treatment parameters and new technical and clinical patient outcome data, the template treatment parameters can dynamically be adapted to new developments and insights into an ablation treatment. Accordingly, the template parameters for treating a patient can be improved and therefore the improved template treatment parameters allow to provide an optimized treatment to a patient.

The treatment parameter providing unit can be, for instance, a storage unit for storing the treatment parameters. Alternatively, the treatment parameter providing unit can be connected to a storage unit storing the treatment parameters. Moreover, the treatment parameter providing unit might also be directly connected to an ablation treatment providing system, wherein the ablation treatment providing system directly provides the treatment parameters to the treatment parameter providing unit. The treatment parameters refer to parameters that are necessary for defining an ablation treatment provided to a patient, for instance, using an ablation treatment providing system, like a microwave ablation system. In a preferred embodiment, the treatment parameters are part of a treatment protocol used for defining the treatment of the patient. Alternatively, the treatment parameters can comprise additional parameters that are not part of a treatment protocol used for treating a patient.

The patient data providing unit can be configured as a storage unit storing the patient data or can be connected to a storage unit storing the patient data. Moreover, the patient data providing unit can be adapted to be directly connected to an ablation treatment providing system, like a high frequency ultrasound ablation system, wherein at least a part of the patient data is directly provided by the ablation treatment providing system to the patient data providing unit. The patient data providing unit is adapted to provide patient data, i.e. data of an individual patient, wherein the patient data comprises technical and clinical patient outcome data. The technical patient outcome data defining the technical outcome of the treatment refer to an outcome that is directly linked to the application of the ablation treatment. For instance, the technical patient outcome data can comprise measurement of the ablated region, knowledge about anatomical structures that were not ablated, or information on a development of a tumor in the ablated region. Preferably, the technical patient outcome data comprises a percentage of tissue ablated in the tumor region, and a percentage of ablated tissue in surrounding organs at risk. Further preferred, the technical patient outcome data comprises a maximum and/or minimum temperature measured or simulated inside the tumor region and/or inside or near organs at risk, and/or dedicated norms calculated from the temperature inside the tumor region or inside or near organs at risk, like a CEM norm, an Arrhenius norm, etc.

The clinical patient outcome data defining the clinical outcome of the treatment refer to a general outcome of the treatment, for instance, to general health data of the patient after the treatment, a survival rate after one, two, or five years after the treatment, the restriction of bodily functions after the treatment, etc. Thus, the patient data allows to evaluate a treatment of a patient with respect to certain treatment goals comprising technical goals, like a specific ablation volume, and clinical goals, like a maintenance of a bodily function. Such treatment goals can also be part of a treatment protocol for the ablation treatment of the patient. Moreover, the patient data can further comprise personal patient data, like age, pre-existing conditions, medications, etc. The patient data corresponds to specific treatment parameters being, for instance, part of a treatment protocol for the patient. Accordingly, the patient data providing unit provides the patient data that corresponds to the treatment parameters provided by the treatment parameter providing unit. Thus, the patient data corresponds to an outcome of the treatment with the treatment parameters provided by the treatment parameter providing unit. The patient data and the treatment parameters can be electronically linked, for instance, through a patient identifier or a coding system.

The template treatment parameter determining unit is adapted to determine template treatment parameters that may form a basis for a template treatment protocol for an ablation treatment. The template treatment parameters define treatment parameters and/or treatment parameter ranges that can be used for a plurality of patients having similar conditions. Template treatment parameters optimize the treatment of a patient and can be applied to a plurality of patients. The template treatment parameters are determined, preferably optimized, based on a relation between any of the treatment parameters, the technical patient outcome data and/or the clinical patient outcome data. Preferably, the template treatment parameter determining unit is adapted to determine the template treatment parameters based on a relation between the treatment parameters and the technical patient outcome data and/or the clinical patient outcome data. For instance, the template treatment parameters can be determined based on a relation between the treatment parameters and the technical patient outcome data to determine, preferably optimize, the template treatment parameters with respect to the technical outcome of the treatment, for instance, with respect to optimizing the ablation region. In another embodiment, the template treatment parameters can be determined based on a relation between the treatment parameters and the clinical patient outcome data, for determining, preferably optimizing, the template treatment parameters with respect to the clinical outcome of the treatment, for instance, with respect to a specific bodily function that should be maintained after the treatment. Moreover, the template treatment parameters can be determined based on a relation between the treatment parameters and both the technical patient outcome data and the clinical patient outcome data to determine, preferably optimize, the template treatment parameters with respect to a technical and clinical outcome of the treatment. Alternatively, for instance, if the same treatment parameters show different patient outcomes, the template treatment parameters can be determined, preferably optimized, based on a relation between the technical patient outcome data and the clinical patient outcome data. In such an embodiment it can be determined if, for instance, additional template treatment parameters are necessary to optimize a technical or clinical outcome of the patient.

Since the template treatment parameters can be determined based on knowledge of the technical and/or clinical outcome of treatments of a plurality of patients with a plurality of treatment parameters, the template treatment parameters ensure that an optimal treatment, with optimal treatment parameters, is provided to the patient. Moreover, the template treatment parameters are determined based on objective measures such that they provide an objective basis for finding treatment parameters for a patient independently of the individual experience of a physician.

The template treatment parameter determining unit can be adapted to determine the template treatment parameters automatically based on the treatment parameters provided by the treatment parameter providing unit and the corresponding patient data provided by the patient data providing unit, for instance, based on a computational analysis of the provided data. Alternatively, the template treatment parameter determining unit can be adapted to determine the template treatment parameters in an interactive process with a user, for instance, a physician. In such an embodiment the template treatment parameter determining unit can be adapted to provide a user with an interface, for instance, to select specific treatment parameters and corresponding patient data based on which the template treatment parameter determining unit should determine the template treatment parameters. Moreover, the template treatment parameter determining unit can in such an embodiment be adapted to display determined template treatment parameters to the user, wherein the user can then, for instance, accept the displayed template treatment parameters as new or updated template treatment parameters or can reject the displayed template treatment parameters, wherein in this case the template treatment parameter determining unit might be adapted to prompt the user to select new treatment parameters and corresponding patient data based on which the template treatment parameters should be determined again.

In an embodiment, the system further comprises an analyzing unit adapted to provide a statistical analysis of the relation between the treatment parameters, the technical patient outcome data and/or the clinical patient outcome data, wherein the template treatment parameter determining unit is adapted to determine the template treatment parameters based on the result of the statistical analysis. Preferably, the statistical analysis comprises correlating treatment parameters with the technical patient outcome data and/or the clinical patient outcome data. Alternatively, the statistical analysis comprises correlating the technical patient outcome data and the clinical patient outcome data. The analyzing unit is then adapted to determine the template treatment parameters based on the results of the correlation. For instance, if a correlation shows that a specific value of a treatment parameter results generally in a better clinical outcome, for instance, results in a general better health of the patient, the template treatment parameter determining unit can be adapted to set the corresponding template treatment parameter to this value. Moreover, if, for instance, a correlation between clinical patient outcome data and technical patient outcome data shows a better clinical outcome for a specific technical outcome, like if a function of an organ is better maintained when the technical outcome refers to a specific percentage of ablation of the organ, new template treatment parameters can be defined that allow to reach this specific technical outcome. The statistical analysis can comprise determining maximum and minimum values, mean values, median values, a mode, a percentile or a quartile of the patient data or the treatment parameters. Preferably, the analyzing unit is further adapted to us advanced statistical algorithms like ANAVO, neural networks, etc.

In an embodiment, the template treatment parameter determining unit is adapted to determining the template treatment parameters based on a relation between the treatment parameters and the technical patient outcome data and/or the clinical patient outcome data. Preferably, in this embodiment the patient data providing unit is further adapted to provide a treatment success indicator indicating the technical and/or clinical success of the treatment of the patient, wherein the statistical analysis comprises correlating treatment parameters with the treatment success indicator. The treatment success indicator indicates the technical and/or clinical success of the treatment of a patient, wherein the treatment success indicator can be calculated based on the technical patient outcome data and/or the clinical patient outcome data. Moreover, the treatment success indicator can indicate the treatment success with respect to a specific treatment goal. For instance, the treatment success indicator can indicate if a specific treatment goal has been reached. For example, if a treatment goal has been to ablate a tumor to 100%, the treatment success indicator can indicate that this goal has been reached to at least 90%, if 90% of the tumor has been ablated. Further, in a preferred embodiment a plurality of treatment goals are defined, wherein the treatment success indicator should indicate the success in reaching the plurality of treatment goals. In this embodiment, during the calculation of the treatment success indicator weights can be provided for weighting the different treatment goals to reflect the importance of each of the different treatment goals. For instance, a treatment goal of ablating a tumor to 100% might have a higher importance and thus the patient data related to this goal is provided with a higher weight than the goal of maintaining a specific bodily function.

In a preferred embodiment the template treatment parameter determining unit can also be adapted to predict a treatment success indicator for the determined template treatment parameters. In this embodiment the template treatment parameter determining unit can be adapted to determine the treatment success indicator for the template treatment parameters based on the treatment success indicators of the treatment parameters provided by the treatment parameter providing unit, i.e. based on the treatment success indicators of the treatment parameters based on which the template treatment parameters are determined. Alternatively, the template treatment parameter determining unit can be configured to provide a machine learning algorithm, like a neural network, to predict a treatment success indicator for the determined template treatment parameters. The machine learning method can be trained, for instance, using the treatment parameters provided by the treatment parameter providing unit and corresponding treatment success indicators. Moreover, the template treatment parameter determining unit can be adapted to optimize the template treatment parameters that are provided based on the relation between the treatment parameters and the technical patient outcome data and/or the clinical patient outcome data, based on the predicted treatment success indicator. For instance, a predetermined predicted treatment success indicator can be used as constraint during the determination of the template treatment parameters. Additionally or alternatively, the template treatment parameter determining unit can be adapted to determine the template treatment parameters in an iterative process, wherein the template treatment parameters are recalculated in each step until a predetermined predicted treatment success indicator is reached.

In an embodiment, the technical patient outcome data comprises ablation measurement data indicative of the ablation region within a patient after an ablation treatment in accordance with treatment parameters for this patient, and/or wherein the clinical patient outcome data comprises health data indicative of one or more health aspects of the patient after the ablation treatment in accordance with treatment parameters for this patient. The ablation measurement data indicative of the ablation region within the patient can comprise, for instance, a volume of the ablated region, a diameter of the ablated region, a position of the ablated region, a position of a boundary of the ablated region, etc. The ablation measurement data can be determined based, for instance, on image data of the ablated region or based on other measurements of the ablated region using, for instance, biopsies, tissue resistance measurements, perfusion measurements, etc. The ablation measurement data can be acquired during the ablation procedure using, for instance, an imaging system like an ultrasound imaging system being part of an ablation treatment providing system, or can be acquired after the ablation procedure using, for instance, an imaging modality like a CT system, an MRI system, an x-ray system, etc. The health data indicative of one or more health aspects of the patient can comprise, for instance, data being indicative of the overall health of a patient, the maintenance of a bodily function of the patient, a survival of the patient after one year or five years, a life quality assessment of the patient, etc. The health data can be acquired through clinical observations and/or measurements after the treatment and/or through patient statements, for instance, acquired during a questionnaire or a patient interview.

In an embodiment, the treatment parameters comprise model parameters for predicting an ablation region in the patient, wherein the statistical analysis further comprises determining a difference between the predicted ablation region and the ablation measurement data of the patient, and correlating the difference with the model parameters to determine a relation between the model parameters and the difference, wherein the template treatment parameters further comprise template model parameters, and wherein the template treatment parameter determining unit is further adapted to determine the template model parameters based on the determined relation. Preferably, the model parameters are part of a numerical model for predicting an ablation region based on the treatment parameters. Preferably, the model parameters comprise at least one of tissue parameters, device parameters and dose parameters. The model parameters can comprise, for instance, tissue parameters like thermal conductivity, tissue density or vessel perfusion, anatomical parameters like the size and location of organs and other anatomical structures like vessels and/or constraints with respect to a thermal dose that should be delivered or to a safety margin surrounding an organ at risk. The model is then adapted to predict, based on the model parameters and the treatment parameters that refer, for instance, to settings of an ablation treatment providing system, an ablation region in the patient. The statistical analysis unit is then adapted to determine a difference between the predicted ablation region and the ablation measurement data of the patient during the statistical analysis. The difference between the predicted ablation region and the ablation measurement data can be any difference, for instance, a difference in the ablated volume, a difference in the location of a boundary of the ablated volume, a different ablation diameter, etc., wherein preferably the difference is indicated by a difference indicator value that is calculated based on the difference in different predicted and measured aspects of the ablation region. The determined difference, for instance, the calculated difference indicator value, can then be correlated with the model parameters used for modelling a prediction of the ablation region, wherein based on this correlation the template treatment parameter determining unit determines, preferably optimizes, template model parameters. For instance, the template treatment parameter determining unit can be adapted to determine based on the correlation that a specific model parameter has to be increased or decreased to provide a better conformity between the predicted ablation region and the measured ablation region. Moreover, the template treatment parameter determining unit can be adapted to use an iterative algorithm to determine the template model parameters, wherein during the iterative algorithms in each step new template model parameters are determined and used as input together with the treatment parameters for predicting a new ablation region, wherein the difference is determined based on the new predicted ablation region and the ablation measurement data, wherein then again the new difference is correlated with the model parameters and for the next step new template treatment parameters are determined based on the new correlation, and so on. The iteration can be aborted if the difference between the predicted ablation region and the measured ablation region is below a predetermined difference, for instance, a predetermined difference indicator value threshold.

In an embodiment, the system further comprises a treatment parameter selection unit adapted to select treatment parameters, wherein the template treatment parameter determining unit is adapted to determine the template treatment parameters based on a relation between any of the selected treatment parameters, the corresponding technical patient outcome data and/or the corresponding clinical patient outcome data. In a preferred embodiment the patient data providing unit is further adapted to provide diagnostic patient data being indicative of a diagnosis of the patient, the diagnostic patient data comprises at least one of volumetric anatomy information, tissue information, affected area information, risk information and lesion classifications, wherein the treatment parameter selection unit is adapted to select the treatment parameters based on any of the diagnostic patient data, technical patient outcome data and/or clinical patient outcome data.

The treatment parameter selection unit can be adapted to select the treatment parameters and the corresponding patient data based, for instance, on the diagnostic patient data of one or more patients such that the template treatment parameters can be determined, preferably optimized, for a specific patient diagnosis, for instance, for a specific tumor in a specific organ of a patient. Additionally or alternatively, the treatment parameter selection unit can be adapted to select the treatment parameters and the corresponding patient data based, for instance, on the clinical outcome data such that the template treatment parameters can be determined, preferably optimized, for a specific clinical outcome, for instance, for a specific function of an organ. Preferably, the treatment parameter selection unit is adapted to provide a user, for instance, a physician, with a user interface providing diagnostic patient data of a plurality of patients, wherein the user can select the diagnostic patient data, the clinical patient outcome data and/or the technical patient outcome data based on which the treatment parameter selection unit should select the treatment parameters. Also the treatment parameter selection unit can provide a user interface on which a user can input specific diagnostic patient data, for instance, a specific lesion classification, specific clinical patient outcome data and/or specific technical patient outcome data, wherein the treatment parameter selection unit is then adapted to search the diagnostic patient data, the clinical patient outcome data and/or the technical patient outcome data provided by the patient data providing unit with respect to this specific diagnostic patient data, specific clinical patient outcome data and/or specific technical patient outcome data, and to select the treatment parameters and patient data corresponding to the respective patient data comprising the respective specific patient data. In a further preferred embodiment, the template treatment parameter determining unit is further adapted to determine the template treatment parameters based on a relation between the selected treatment parameters, the diagnostic patient data, the technical patient outcome data and the clinical patient outcome data. Preferably, the same statistical and analytical methods as already described above are used to determine the relation between the selected treatment parameters, the diagnostic patient data, the technical patient outcome data and the clinical patient outcome data and to determine the template treatment parameters based on this relation. Since the diagnostic patient data is taken into account in this embodiment, the specific situation of each patient before the treatment can be taken into account such that a relation that can only be determined for specific diagnostic patient data can be considered during the determination of the template treatment parameters. This allows to determine, preferably optimize, the template treatment parameters such that they are applicable for a plurality of patients having a same or a similar medical condition.

In an embodiment, the treatment parameters comprise decision parameters defining a clinical decision based on a diagnostic finding of a patient and device setting parameters defining the device setting of an ablation device during the ablation treatment of the patient. The decision parameters preferably comprise ablation goals, like a percentage of a tumor that should be ablated, a maximal percentage of an organ at risk that shout be ablated and condition parameters like a tumor size, a lesion classification, a tumor location, etc., wherein the clinical decision should be based on these condition parameters. Moreover, the decision parameters can comprise a link between the decision parameters and the device setting parameters such that, for instance, a specific tumor location, a tumor size or a specific percentage of the tumor that should be ablated refers to specific device setting parameters. Preferably, the device setting parameters comprise at least one of device energy settings, ablation timing parameters, device location parameters and delivered dose parameters.

In a further aspect of the present invention, a method for assisting in providing template treatment parameters for an ablation treatment is presented, wherein the method comprises the steps of a) providing treatment parameters, wherein the treatment parameters comprise parameters defining a treatment of a patient, b) providing patient data of a plurality of patients treated with the treatment parameters, wherein the patient data comprises technical patient outcome data defining the technical outcome of the treatment and clinical patient outcome data defining the clinical outcome of the treatment, c) determining template treatment parameters, wherein the template treatment parameters are determined based on a relation between any of the treatment parameters, the technical patient outcome data and/or the clinical patient outcome data.

In a further aspect of the present invention, a computer program for assisting in providing template treatment parameters for ablation treatment is presented, wherein the computer program comprises program code means for causing the system of claim 1 to carry out the steps of the method as defined in claim 14 when the computer program is executed on the system.

It shall be understood that the system of claim 1, the method of claim 14 and the computer program of claim 15 for assisting in providing template treatment parameters for ablation treatment have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for assisting in providing template treatment parameters for ablation treatment,
Fig. 2 shows schematically and exemplarily a function of such a system,
Fig. 3 shows schematically and exemplarily a display provided to a user of such a system, and
Fig. 4 shows a flowchart exemplarily illustrating an embodiment of a method for assisting in providing template treatment parameters for ablation treatment.

### DETAILED DESCRIPTION OF EMBODIMENTS

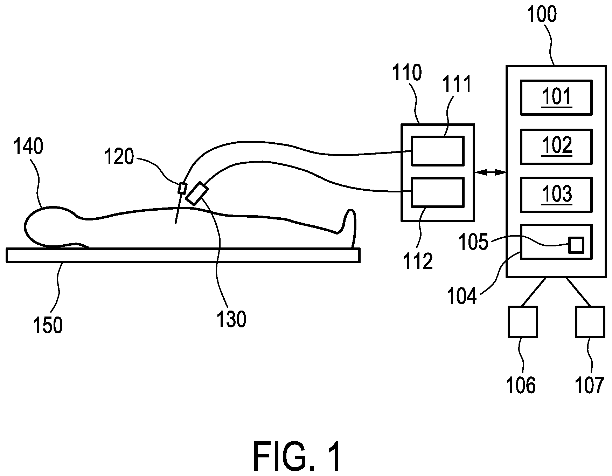

Fig. 1 shows schematically and exemplarily an embodiment of a system 100 for assisting in providing template treatment parameters for ablation treatment. In this embodiment, the system 100 comprises a treatment parameter providing unit 101, a patient data providing unit 102, a treatment parameter selection unit 103 and a template treatment parameter determining unit 104. In this exemplary embodiment, the system 100 further interacts with an ablation treatment providing system 110 comprising an ablation device control unit 111 and an imaging control unit 112. The ablation device control unit 111 controls an ablation device 120 which can be, for instance, a high frequency ultrasound device. The imaging control unit 112 controls an imaging device 130 which can be, for instance, an ultrasound imaging device. The ablation device 120 is adapted to ablate a region within a patient 140 lying on a patient support 150 while the imaging device 130 is adapted to provide images of the ablated region during and/or after the ablation procedure.

In this embodiment the system 100 and the ablation treatment providing system 110 interact such that the ablation treatment providing unit 110 provides treatment parameters used during the treatment of the patient 140 to the treatment parameter providing unit 101, wherein the treatment parameters comprise, for instance, device settings used by the ablation device controlling unit 111 for controlling the ablation device 120. Further, the treatment parameters can be part of a treatment protocol for defining the treatment of the patient 140, wherein in this case the ablation treatment providing unit provides the treatment protocol to the treatment parameter providing unit 101. In this embodiment, the interaction also comprises providing patient data comprising technical outcome patient data for the ablation treatment to the patient data providing unit 102. The technical patient outcome data are determined, for instance, based on images of the ablated region provided by the imaging device 130 and processed by the imaging control unit 112 to determine the technical patient outcome data. Alternatively, the imaging control unit 112 can provide the image data acquired by the imaging device 130 to the patient data providing unit 102, wherein then the patient data providing unit 102 is adapted to determine the technical patient outcome data based on the provided image data. The technical patient outcome data can comprise a determined ablation region volume and a determined location of a boundary of the ablation region.

The treatment parameter providing unit 101 of the system 100 is in this embodiment adapted to provide treatment parameters of a plurality of patients treated with the ablation treatment providing system 110. The patient data providing unit 102 is adapted to provide the technical patient outcome data of the respective patients for which the treatment parameters are provided by the treatment parameter providing unit 101. The treatment parameter selection unit 103 is adapted to select the treatment parameters and the corresponding patient data provided by the treatment parameter providing unit 101 and the patient data providing unit 102, respectively.

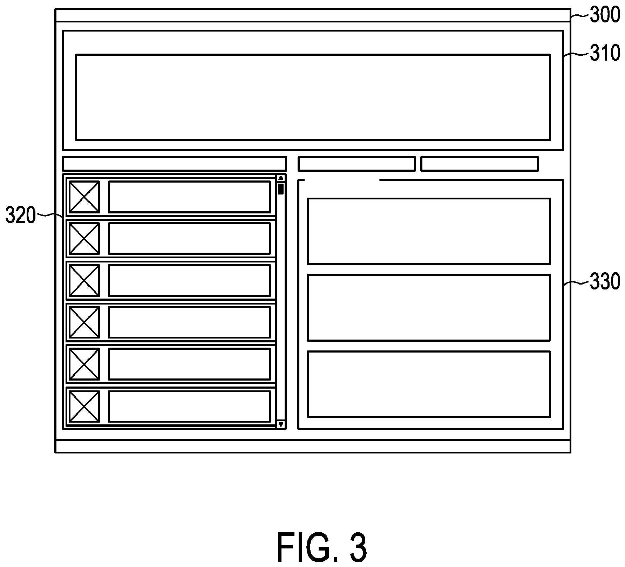

In an embodiment, the treatment parameter selection unit 103 is adapted to provide a user interface to a user, for instance, a physician, via a display 106 connected to the system 100. The user can interact with the user interface 300 using, for instance, an input device 107, like a mouse or keyboard. The user interface can comprise, for instance, a display as shown in Fig. 3. The user interface 300 exemplarily shown in Fig. 3 comprises a template treatment protocol information box 310 comprising information on template treatment parameters of a currently used template treatment protocol used as basis for determining a treatment protocol comprising treatment parameters for a patient. Moreover, the template treatment protocol information box 310 could also provide the treatment parameters of a new or updated template treatment protocol comprising new or updated template treatment parameters. Further, the user interface 300 comprises a retrospective cases section 320 showing a short summary of previous ablation treatment cases. The summary can comprise, for instance, diagnostic patient data like an image of the tumor region or a classification of the tumor, important treatment parameters like device settings, for instance, a provided ablation dose or a provided ablation power, and/or a treatment success indicator indicating the success of the provided treatment. Moreover, the user interface 300 comprises a further constraint selection window 330 in which a user can select further constraints for the selection of the shown cases in section 320. For instance, the user might be provided in the constraint selection window 330 with selection options like which ablation device is used for the ablation, which organ is concerned, how a lesion is classified, etc. Based on this user interface 300 the user can then select the constraints, wherein the treatment parameter selection unit 103 then selects the cases shown to a user in the retrospective cases section 320. The user can then accept, for instance, the cases shown in the retrospective cases section 320 as the cases based on which the template treatment parameters should be determined or can amend the selection provided by the treatment parameter selection unit 103. Based on the case selection by the user the treatment parameters selection unit 103 selects the treatment parameters corresponding to these cases and also the patient data corresponding to these cases.

In this embodiment, the template treatment parameter determining unit 104 comprises an analyzing unit 105, wherein the analyzing unit 105 is adapted to provide a statistical analysis of a relation between the selected treatment parameters, the corresponding technical patient outcome data and/or the corresponding clinical patient outcome data. Based on the result of the analysis, for instance, based on a relation between the treatment parameters and the patient data, the template treatment parameter determining unit 104 determines, preferably optimizes, new template treatment parameters.

The template treatment parameters determined by the template treatment parameter determining unit 104 can then be used, for instance, as basis for determining treatment parameters for a patient. For instance, the template treatment parameters can be used as treatment parameters for the patient. The respective determined treatment parameters, which are determined based on the template treatment parameters, can then be provided as input to the ablation device control unit 111 of the ablation treatment providing system 110 to provide an optimized ablation treatment to the patient.

An example for an analysis provided by the analyzing unit 105 and the determination of template treatment parameters by the template treatment parameter determining unit 104 is given in the following. For this example, it is assumed that in a hospital 100 patients have been treated with the same treatment protocol for thermal ablation in the liver. For 5 out of these 100, side effects in relation to the lungs occurred. These occurrences all involved cases in which the tumor was located close to the diaphragm. Because of that, the treating physicians would like to define a template treatment protocol comprising treatment parameters for treating tumors that are close to the diaphragm, based on the historic data. Firstly, the treatment data selection unit 103 selects all cases in which the tumor was located close to the diaphragm (within 3 cm). This results in 18 out of 100 cases, including the 5 cases in which side effects occurred. In 12 cases the clinical patient outcome data shows that no side effects occurred. In this example the cases without side effect, i.e. with postive clinical outcome, comprise as technical patient outcome data the following:

| Case | portion of target ablated | portion of liver ablated | portion of diaphragm ablated |
|---|---|---|---|
| 1 | 100% | 2% | 2% |
| 2 | 100% | 2% | 4% |
| 3 | 99% | 2% | 3% |
| 4 | 98% | 3% | 2% |
| 5 | 100% | 2% | 2% |
| 6 | 99% | 3% | 4% |
| 7 | 100% | 2% | 3% |
| 8 | 99% | 3% | 2% |
| 9 | 100% | 2% | 2% |
| 10 | 100% | 2% | 4% |
| 11 | 100% | 2% | 3% |
| 12 | 100% | 3% | 2% |

Since all cases comprise the same treatment parameters but refer to different clinical outcomes, a new additional template treatment parameter might be necessary to optimize the clinical outcome for the treatment parameters used. Therefore, the analyzing unit 105 analyzes a relation between the clinical patient outcome data and the technical patient outcome data. In this case the analysis comprises determining the maximum or minimum of the technical patient outcome data that is related to a positive clinical outcome, i.e. to clinical patient outcome data showing that no side effects occurred. Based on these determined maxima and minima the template treatment parameter determining unit 104 determines in this case decision parameters, in particular goal parameters, as additional template treatment parameters, wherein these template treatment parameters comprise, for instance, that more than 98% of the target should be ablated, no more than 3% of the liver should be ablated, and no more than 4% of the diaphragm should be ablated to avoid the occurrence of side effects in relation with the lungs. From these goal parameters further new template treatment parameters referring to device settings are determined, for instance, by adjusting the device settings of the treatment parameters used during the treatment of the patients to reflect the new additional treatment goals. The new template treatment parameters can then be sent to the ablation treatment providing system 110.

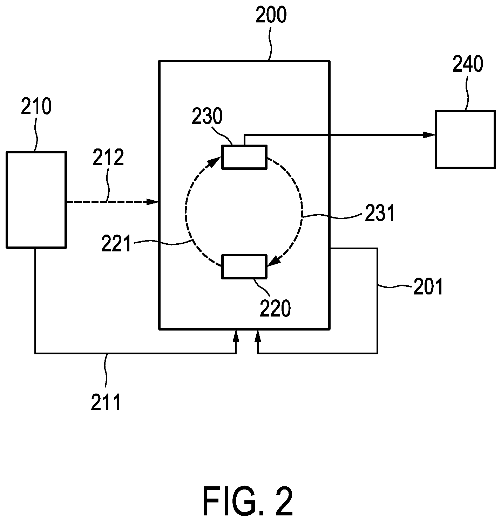

A further detailed example of a function of the system 100, in particular of the input and output of the system 100, is given with respect to Fig. 2. Fig. 2 shows schematically an exemplary template treatment parameter determination processes 200 of system 100. An input 210 comprising treatment parameters, patient data and optionally diagnostic patient data is provided as input into the process 200 as indicated by the arrow 211. Moreover, in an evaluation process 220, for the input treatment parameters and/or already existing template treatment parameters a treatment success is determined, for instance, by determining a treatment success indicator based on the input patient data. Based on the results of the evaluation process 220 and the input 210, as indicated by arrows 212 and 221, in a determination process 230 new or updated template treatment parameters are determined. These new or updated template treatment parameters can then again be used as input for an evaluation process 220, as indicated by the circular arrow 231, for instance, by predicting a treatment success indicator for the new template treatment parameters. Based on an evaluation of the new template treatment parameters and/or based on new or additional input cases, the process 230 can again determine or update template treatment parameters. Optionally, during the process 200, additional information like dose and toxicity calculations can be considered, as indicated by the arrow 201. For instance, based on the new template treatment parameters a delivered dose can be predicted and this prediction can be considered when determining the new template treatment parameters. If after an evaluation of the new or updated template treatment parameters, it is indicated that the new or updated template treatment parameters are acceptable with respect to a predetermined criterion, for instance, due to providing a better treatment success indicator than the previous template treatment parameters, the process 200 provides as output data 240 the new template treatment parameters. The new template treatment parameters can then be incorporated, for instance, in a new template treatment protocol for a specific ablation method.

Although in the above described embodiment a treatment parameter selection unit is described, in other embodiments of the system the treatment parameter selection unit can be omitted and the template treatment parameters can be determined based on all treatment parameters and corresponding patient data provided by the treatment parameter providing unit and the patient data providing unit. Moreover, although in the above embodiment a specific exemplary user interface for the selection of cases for selecting the treatment parameters provided by the treatment parameter selection unit is shown, in other embodiments the selection unit can provide a completely different user interface or no user interface at all. For instance, the treatment parameter selection unit can also select the treatment parameter without user interaction, based on a predetermined criterion.

Although in the above example additional template treatment parameters are determined based on a relation between the clinical patient outcome data and the technical patient outcome data, in other embodiments the template treatment parameters can also be determined based on a relation between the treatment parameters and the clinical patient outcome data and/or the technical patient outcome data, or further based on the diagnostic patient data using correspondingly adapted methods as described above.

Although in the above embodiment the system directly interacts with an ablation treatment providing system, in other embodiments the system does not directly interact. For instance, the ablation treatment providing system can provide the treatment parameters and/or patient data to a storage unit, wherein the treatment parameter providing unit and the patient data providing unit are connected to this storage unit at some point to provide the treatment parameters and the patient data.

Moreover, in a further embodiment the system can comprise a numerical modelling unit, wherein the numerical modelling unit is adapted to predict an ablation region in the patient based on treatment parameters and model parameters. In such an embodiment the statistical analysis unit can further be adapted to determine a difference between the predicted ablation region and the ablation region measured during or after the ablation, for instance, by an imaging device. This difference can then be correlated to the model parameters used in the numerical model, wherein based on this correlation template model parameters can be determined that allow for a better prediction of the ablation region, i.e. that allow to minimize the difference between the predicted ablation region and the measured ablation region.

Although in the above embodiment a high frequency ultrasound ablation device has been used, in other embodiments also other ablation devices can be provided, for instance radio frequency ablation devices, microwave ablation devices, cryo devices, etc. Although in the above embodiment an ultrasound imaging device is used for providing ablation measurement data, in other embodiments other imaging devices can be used, for instance, x-ray systems, CT systems, MRI systems etc. Moreover, other measurement devices and method can be used to provide technical outcome data for a patient, for instance, perfusion measurement methods, tissue resistance measurement methods, temperature sensors like thermos-couples, etc.

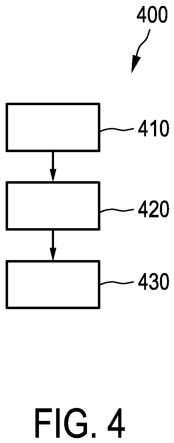

Fig. 4 shows a flowchart exemplarily illustrating an embodiment of a method for assisting in providing template treatment parameters for an ablation treatment. The method 400 comprises a first step 410 of providing treatment parameters defining a treatment of a patient. Further, the method 400 comprises a step 420 of providing patient data of a plurality of patients treated with the treatment parameters, wherein the patient data comprises technical patient outcome data and/or clinical patient outcome data. The technical patient outcome data defines a technical outcome of the treatment, for instance, the size of an ablated region, the presence or absence of ablation in an anatomical structure, a provided ablation dose, etc. The clinical patient outcome data defines the clinical outcome of the treatment, for instance, a survival rate of a patient during the five years following the treatment, the maintenance of a bodily function, etc. The method 400 comprises further a step 430 of determining template treatment parameters based on a relation between any of the treatment parameters, the technical patient outcome data and/or the clinical patient outcome data.

Minimally invasive interventions for cancer treatment allow for therapy delivery while minimizing the damage of surrounding healthy tissue. Thermal ablation, using heat, for instance radio frequency ablation, microwave ablation, high frequency ultrasound ablation, or cold, for instance, cryoablation, is increasingly used as focal therapy for small tumors, for instance, in the liver, kidney, lung or prostate.

The execution of a thermal ablation therapy involves several steps, starting with selecting or defining treatment parameters that can be part of a treatment protocol, followed by planning the therapy and executing the therapy. Several systems have been proposed for the purpose of planning and guiding a physician through such a therapy, wherein the systems are typically specific towards one ablation device. However, at the moment no standardized treatment protocols comprising standardized treatment parameters are known for ablation therapy and the execution parameters, i.e. the treatment parameters, for an ablation therapy are typically defined on an ad hoc basis by physicians. This is opposed to the protocols used in radiation therapy based approaches which are typically part of a hospital quality control and are based on established medical guidelines.

When defining treatment parameters of a treatment protocol for ablation therapy, a plurality of considerations have to be taken into account by a physician, for instance, target thermal dose constraints to be delivered to a target, constraints to a lesion or organ and to the surrounding organs at risk, organ/lesion margins, organ tissue properties including thermal conductivity, vessel perfusion, tissue density, device or type of device used for ablation, like if a cryo or microwave device is used etc. Information that can be used for defining treatment parameters of a treatment protocol can comprise, for instance, imaging data, anatomical definitions like organ locality, whether an organ is a target, whether an organ can be punctured, a dosed engine or thermal dose delivered by a device as provided by a manufacturer of the device, etc.

If a template treatment protocol comprising template treatment parameters should be provided that should be used for a plurality of patient cases, the template treatment parameters of such a template treatment protocol can be tuned to a hospital to maximize the efficiency of the treatment and provide a better quality assurance of the treatment. However, defining such template treatment parameters is a complex task and the hospital needs, for instance, to understand the treatment parameters that have been used during an ablation intervention and their relation to a patient outcome while taking into account inherent uncertainties at each step like uncertainties in planning, instrument insertion, tissue properties and delivered dose.

In this invention it is proposed to apply descriptive statistical methods for revealing correlations between the treatment parameters and patient data comprising technical and/or clinical patient outcome data, and to determine, tune or refine template treatment parameters of a template treatment protocol based on revealed correlations between the treatment parameters and the patient data.

Refining the treatment parameters based on cases of previously treated patients typically take these elements implicitly into account based on the experience of a physician. In this invention, to the contrary, it is proposed to provide a tool to help a physician to define, refine or tune template treatment parameters by providing the required information, i.e. patient data and treatment parameters, and by providing a statistical analysis of the respective correlations between the patient data and treatment data.

Such a system can, for instance, comprise an interactive tool that allows as input data from previous cases diagnostic patient data, like volumetric anatomy information like organ delineations, a planned dose, device placement information, which can be obtained either via specific imaging techniques revealing ablated areas like contrast imaging or a combination of device placement information synchronized with device activation information, etc. Moreover, the interactive tool can allow as input treatment parameters referring to the planning constraints or model parameters, like dose constraints, tissue parameters, whether an organ is a target, whether an organ can be punctured, organs at risk to take into account, or margins to add to each organ, etc. Further, the input to the interactive tool can comprise patient data comprising outcome information, i.e. technical and clinical patient outcome data, like a delivered dose that can be measured via a contrast agent imaging, side effects, a comparative display of a delivered thermal dose and a measured ablation zone. Moreover, such a system can further comprise tools for reporting treatment parameters referring to device parameters, like an ablation power or duration setting during an ablation. Furthermore, the system can also provide a storage for storing data with respect to technical patient data referring to a relation between a delivered thermal dose and a measured ablation zone. Also a storage can be provided for storing a relation between used treatment parameters and a measured ablation zone. Additionally, a training model to derive a relation between a delivered thermal dose and an ablation zone, and a training model to derive a relation between used treatment parameters and technical patient outcome data referring to a measured ablation zone might be provided. Such a system can further comprise a feedback providing unit for providing a feedback of the success of determined template treatment parameters in a therapy application.

A provided retrospective analysis of the technical patient outcome data referring, for instance, to a thermal dose delivered using specific treatment parameters based on each treatment can provide insights on a definition or update of template treatment parameters of a template treatment protocol suitable for the institutional way of working with specific devices. Since the currently used treatment parameters are not well defined, a wide variation of treatment parameters are available to provide ground for a statistical analysis.

It is further proposed that a system for assisting in providing template treatment parameters for ablation treatment may further comprise, for instance, a support module. Such a support module might be adapted to provide, based on the provided treatment parameters and patient data, a batch processing of multi-intraoperative image/volume data sets, a calculation of a total thermal dose based on determined device locations, a segmentation and displaying of an ablation zone, a comparative displaying of a thermal dose related to an ablation zone, a controlling and determination of a device placement and/or a positioning of ablation devices and an assignment of an ablation power and duration for each case. The system may further also comprise a thermal energy simulation module for computing a thermal dose or tissue toxicity, wherein the simulation module can be adapted, for instance, to predefine geometrical regions in an anatomical image, wherein the predefined anatomical regions are provided, for instance, by an ablation device manufacturer, and/or to simulate temperature effects on the human body. Accordingly, the thermal energy simulation module can also comprise a numerical module for simulating, i.e. predicting a thermal ablation region within a patient.

Moreover, the system may further comprise units for storing/retrieving treatment parameters like ablation device settings, power settings, duration settings of an ablation device, or a planned thermal dose region, i.e. ablation region. Further, units might be provided for storing/retrieving patient data like a delivered thermal dose region determined from imaging data in which the actual ablation region is visible, for instance, using contrast agent imaging. Further, the system may comprise an analyzing unit, wherein the analyzing unit is adapted to statistically analyze the relation between treatment parameters and patient data. For instance, the analyzing unit can be adapted to derive relational parameters based on a planned versus a delivered ablation region, to derive relational parameters based on treatment parameters versus a measured ablation region. The determined relational parameters can then be used during the determination of the template treatment parameters, for instance, to ensure that a predicted ablation region better matches a measured ablated region.

Moreover, a treatment protocol comprising treatment parameters can be refined or updated, wherein during this refinement or updating it can be determined how changes in the treatment parameters affect a prediction of a treatment outcome for already existing patient cases. This can be used, for instance, to fine-tune model parameters, like tissue parameters, or the prediction of a thermal dose delivered by an available device, or to provide parameters for a better alignment with an actual ablation zone in a patient, for example when a different tissue area is treated.

Further, a sensitivity analysis of a treatment protocol comprising treatment parameters can be performed by a system assisting in providing template treatment parameters for ablation treatment to see how certain treatment parameters influence clinical goals.

Other variations to the disclosed embodiments can be understood and effected by those skilled in the art and practicing the planned invention from the study of the drawings, the disclosure and the appendant claims.

In the claims, the word "comprising" does not exclude other elements or steps, and the indefinite article "a" or "an" does not exclude a plurality.

A single unit or device may fulfil the functions of several items recited in the claims. The mere fact that certain measures are recited in mutual different dependent claims does not indicate that a combination of these measures cannot be used to advantage.

Procedures like the providing of treatment parameters or the providing of template treatment parameters performed by one or several units or devices can be performed by any other number of units or devices. The procedures and/or the operations of the system can be implemented as program code means of a computer program and/or as dedicated hardware. A computer program may be stored/distributed in a suitable medium, such as any optical storage medium or solid state medium, supplied together with or as part of other hardware, but might also be distributed in other forms, such as via the internet or other wired or wireless communication systems.

Any reference signs in the claims should not be construed as limiting the scope.

The present invention refers to providing a system allowing to provide improved template treatment parameters for ablation treatment allowing an optimized ablation treatment. A system for assisting in providing template treatment parameters is presented. The system comprises a treatment parameter providing unit providing treatment parameters, a patient data providing unit providing technical patient outcome data and/or clinical patient outcome data, a template treatment parameter determining unit for determining template treatment parameters based on a relation between any of the treatment parameters, the technical patient outcome data and/or the clinical patient outcome data. Thus, the template treatment parameters can be optimized in accordance with the technical and/or clinical outcome of the treatment applied using the treatment parameters. Accordingly, the template parameters for treating a patient can be improved and the improved template treatment parameters allow to provide an optimized treatment to a patient.

## Claims

1. System for assisting in providing template treatment parameters for ablation treatment, comprising:
- a treatment parameter providing unit (101) adapted to provide treatment parameters, wherein the treatment parameters comprise parameters defining an ablation treatment of a patient;
- a patient data providing unit (102) adapted to provide patient data of a plurality of patients treated with the treatment parameters, wherein the patient data comprises technical patient outcome data defining the technical outcome of the treatment and/or clinical patient outcome data defining the clinical outcome of the treatment;
- a template treatment parameter determination unit (104) adapted to determine template treatment parameters, wherein the template treatment parameters are determined based on a relation between any of the treatment parameters, the technical patient outcome data and/or the clinical patient outcome data.

2. System according to claim 1, wherein the system further comprises an analyzing unit (105) adapted to provide a statistical analysis of the relation between the treatment parameters, the technical patient outcome data and/or the clinical patient outcome data, wherein the template treatment parameter determining unit (104) is adapted to determine the template treatment parameters based on the result of the statistical analysis.

3. System according to claim 2, wherein the statistical analysis comprises correlating treatment parameters, the technical patient outcome data and/or the clinical patient outcome data.

4. System according to any of claims 1 to 3, wherein the template treatment parameter determining unit (104) is adapted to determining the template treatment parameters based on a relation between the treatment parameters and the technical patient outcome data and/or the clinical patient outcome data.

5. System according to claim 4, wherein the patient data providing unit (102) is further adapted to provide a treatment success indicator indicating the technical and/or clinical success of the treatment of the patient, wherein the statistical analysis comprises correlating treatment parameters with the treatment success indicator.

6. System according to claim 5, wherein the treatment success indicator is based on the technical and/or clinical patient outcome data.

7. System according to any of claims 2 to 6, wherein the technical patient outcome data comprises ablation measurement data indicative of the ablation region within a patient after an ablation treatment in accordance with treatment parameters for this patient, and/or wherein the clinical patient outcome data comprises health data indicative of one or more health aspects of the patient after the ablation treatment in accordance with treatment parameters for this patient.

8. System according to claim 7, wherein the treatment parameters comprise model parameters for predicting an ablation region in the patient, wherein the statistical analysis further comprises determining a difference between the predicted ablation region and the ablation measurement data of the patient, and correlating the difference with the model parameters to determine a relation between the model parameters and the difference, wherein the template treatment parameters further comprise template model parameters, and wherein the template treatment parameter determining unit (101) is further adapted to determine the template model parameters based on the determined relation.

9. System according to claim 8, wherein the model parameters comprise at least one of tissue parameters, device parameters and dose parameters.

10. System according to any of claims 1 to 9, wherein the system (100) further comprises a treatment parameter selection unit (103) adapted to select treatment parameters, and wherein the template treatment parameter determining unit (104) is adapted to determine the template treatment parameters based on a relation between any of the selected treatment parameters, the corresponding technical patient outcome data and/or the corresponding clinical patient outcome data.

11. System according to any of claims 1 to 10, wherein the patient data providing unit (102) is further adapted to provide diagnostic patient data being indicative of a diagnosis of the patient, the diagnostic patient data comprises at least one of volumetric anatomy information, tissue information, affected area information, risk information and lesion classifications, wherein the treatment parameter selection unit (103) is adapted to select the treatment parameters based on any of the diagnostic patient data, technical patient outcome data and/or clinical patient outcome data.

12. System according to any of claims 1 to 11, wherein the treatment parameters comprise decision parameters defining a clinical decision based on a diagnostic finding of a patient and device setting parameters defining the device setting of an ablation device during the ablation treatment of the patient.

13. System according to claim 12, wherein the device setting parameters comprise at least one of device energy settings, ablation timing parameters, device location parameters and delivered dose parameters.

14. Method for assisting in providing template treatment parameters for an ablation treatment, comprising the steps of:
- providing (410) treatment parameters, wherein the treatment parameters comprise parameters defining a treatment of a patient;
- providing (420) patient data of a plurality of patients treated with the treatment parameters, wherein the patient data comprises technical patient outcome data defining the technical outcome of the treatment and clinical patient outcome data defining the clinical outcome of the treatment;
- determining (430) template treatment parameters, wherein the template treatment parameters are determined based on a relation between any of the treatment parameters, the technical patient outcome data and/or the clinical patient outcome data.

15. Computer program for assisting in providing template treatment parameters for ablation treatment, the computer program comprising program code means for causing the system of claim 1 to carry out the steps of the method as defined in claim 14 when the computer program is executed on the system.
